# EUROPEAN PATENT APPLICATION

(11) **EP 3 124 080 A1**
(43) Date of publication of application: **01.02.2017**
(21) Application number: 15178637.3
(22) Date of filing: 28.07.2015
(51) Int. Cl.: A61Q 19/06, A61P 3/04, A61K 31/575, A61K 9/00, A61K 9/107

(54) **SEMISYNTHETIC BILE ACIDS FOR INJECTION LIPOLYSIS**

(71) Applicant: Merz Pharma GmbH & Co. KGaA, 60318 Frankfurt am Main (DE)
(72) Inventor: Abel, Ulrich, 61350 Bad Homburg (DE); Abts, Harry Frank, 61440 Oberursel (DE); Hengl, Thomas, 60437 Frankfurt (DE); Parsons, Christopher Graham Raphael, 61130 Nidderau (DE); Danysz, Wojciech, 61130 Nidderau (DE)
(74) Representative: Jacobi, Markus Alexander

(57) **Abstract**

The present invention relates to a composition comprising a compound or a pharmaceutically acceptable salt thereof having a structure according to formula (I) 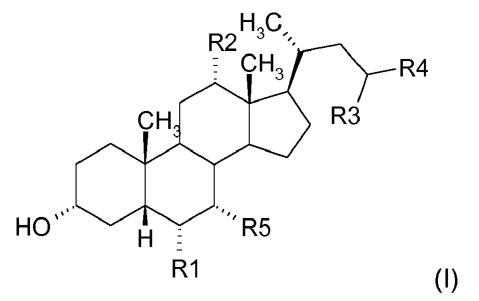 wherein R1 is an unsubstituted or substituted C₁₋₄ alkyl residue and residues R2-R5 are defined as specified in the description and at least one further component C for use in a method for local treating or preventing a condition associated with an undesired local subcutaneous fat deposit in a mammal, wherein said compound is injected into said local subcutaneous fat deposit aiming for its reduction. Further, the present invention relates to the cosmetic use of such composition.

## Description

The present invention relates to semisynthetic bile acids for injection lipolysis and to a composition comprising a compound or a pharmaceutically acceptable salt thereof having a structure according to formula (I) 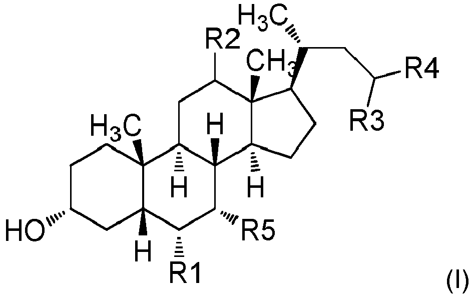 wherein R1 is an unsubstituted or substituted C₁₋₄ alkyl residue and residues R2 to R5 are defined as specified in the description, and at least one further component C for use in a method for local treating or preventing a condition associated with an undesired local subcutaneous fat deposit in a mammal, wherein said compound is injected into said local subcutaneous fat deposit aiming for reduction of local subcutaneous fat. Further, the present invention relates to the medicinal aesthetic and cosmetic use of such composition.

Body contouring by means of injection lipolysis plays an increasing role in a number of pharmaceutical and cosmetic applications. Herein, a composition comprising such agent is directly injected into one or more local subcutaneous fat deposit(s) (depot(s)) of interest. For this purpose, the composition comprising such agent is directly injected into the respective target fat-tissue in the mammal's body *in situ.* It is thereby intended to reduce such one or more local subcutaneous fat deposit(s) in size. This is typically achieved by a partly or complete degradation of the respective adipose tissue (fatty tissue) by means of a disintegration of the adipocytes (fat cells) in the local subcutaneous fat deposit(s) of interest.

The released cellular fragments are typically removed by the physiologic mechanisms such as by means of macrophages.

Several natural bile acids such as, e.g., deoxycholate (DC), chenodeoxycholic acid (CDCA), ursodeoxycholate (UDCA) and lithocholic acid (LCA) are used for cosmetic applications in the art. Additionally, phosphatidylcholine (PC) may be used for this purpose. These compounds are described suitable for reducing adipose tissue by means of injection lipolysis.

However, these compounds used in the art do not have the desired properties such as effectiveness in treating and preventing undesired fat accumulations.

Therefore, there is an unmet need for compounds and compositions that can be effectively used for injection lipolysis and, concomitantly, cause less severe side effects. In particular for body contouring, i.e., for shaping the mammal's outer appearance (for pharmaceutical and/or cosmetic purposes) mammal's compliance is desired to be significantly improved. Therefore, there is a need for compounds and compositions that bear a higher selectivity to adipose tissue and are well-tolerably by the mammal's body.

A variety of compounds like several semisynthetic bile acid analogs have been described as bearing agonistic activity on farnesoid X receptor (FXR) and/or Takeda G protein-coupled receptor 5 (TGR5). Such FXR agonists and/or TGR5 agonists are known to have valuable pharmaceutical activities associated with systemic and cellular lipid metabolism (Lundquist, 2010, J. Med. Chem. 53:1774-1787; Mencarelli, 2013, Nutrition, Metabolism and Cardiovascular Diseases 23:94-101; Stanimirov, 2012, Acta Gastro-Enterologica Belgica 75:389-398; Teodoro, 2011, Trends Endocrinol Metab. 22(11):458-466; Pellicciari, 2007, J. Med. Chem. 50:4265-4268; Kawamata, 2003, J. Biol. Chem. 278(11):9435-9440; Pellicciari, 2005, J. Med. Chem. 48(17):5383-5403; and Watanabe, 2006, Nature 439:483-489).

Such agonists are in principle pharmaceutically active in *in vitro* tests in cell culture and suggested for systemic applications, mostly by oral uptake of the compounds. It was however neither described nor suggested to use such compounds for removing or decreasing fat deposits, in particular not locally administering a composition in the patient *in situ.*

Surprisingly, it has been found that instead of natural bile acids, certain types of semisynthetic compounds may be used for injection into an undesired local subcutaneous fat deposit *(in situ)*, thereby reducing the fat deposit in size. The compositions used are well-tolerated by the mammal's body.

In a first aspect, the present invention relates to a composition comprising a compound or a pharmaceutically acceptable salt thereof having a structure according to formula (I) 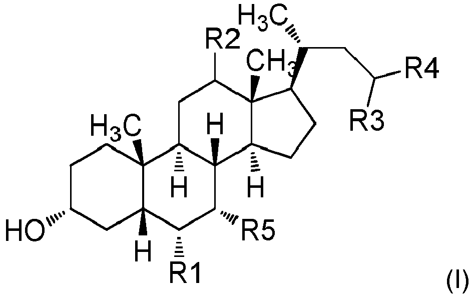 wherein:
R1 is a C₁₋₄ alkyl residue;
R2 is hydrogen or -OH;
R3 is hydrogen or a C₁₋₄ alkyl residue;
R4 is selected from the group consisting of a charged group, preferably a negatively charged group, in particular -COO⁻X⁺ or -OSO₃⁻X⁺, wherein X⁺ is a proton or a pharmaceutically acceptable cation, a conjugate of a carboxylic group, in particular a glycin or taurine conjugate, and an isostere of -COO⁻X⁺ (-COOX); and
R5 is hydrogen or -OH;
   and at least one further component C
for use in a method for local treating or preventing a condition associated with an undesired local subcutaneous fat deposit in a mammal, wherein said compound is injected into said local subcutaneous fat deposit.

The present invention relates to a method for local treating or preventing a condition associated with an undesired local subcutaneous fat deposit in a mammal, wherein a composition comprising a compound of formula (I) or a pharmaceutically acceptable salt thereof and at least one further component C is injected into said local subcutaneous fat deposit (*in situ*).

The composition of the present invention results in the elimination of adipocytes by cell lysis (adipocytolysis). In part the elimination of adipocytes is reached by compound induced apoptosis (lipocytolysis). Compound mediated pharmacological induction of endogenous lipolysis delivers a further supportive activity: 1. The size of non-eliminated adipocytes is reduced by the release of stored fat. 2. The uptake of fat from destroyed fat-cells by surrounding fat cells is reduced.

When used in the context of the present invention, i.e., when injected into an undesired subcutaneous local fat deposit (*in situ*), the semisynthetic bile acid analogs according to the composition of the present invention are unexpectedly more effective than the respective natural bile acids although the physicochemical properties are rather similar (cf., Roda, 2014, J Pharmacol Exp Ther. 350(1):56-68). The effect may therefore be understood as an unexpectedly synergistic combination of physicochemical and physiological activities. Apparently, the composition of the present invention may induce apoptosis (lipocytolysis).

A pharmaceutically acceptable salt may be understood in the broadest sense as any salt that bears a comparably low toxicity and is acceptable for pharmaceutical purposes. Exemplarily but not limiting, a pharmaceutically acceptable salt may comprise a cation selected from the group consisting of an alkali metal (in particular, Na⁺ and/or K⁺), a proton (i.e., H⁺), an alkaline earth metal (in particular, Mg²⁺ and/or Ca²⁺), ammonium (NH₄⁺), Fe²⁺, Fe³⁺, Zn²⁺, Sn²⁺, and an organic amine cation.

As the composition according to the present invention may optionally also comprise one or more salts, it may also comprise anions like, exemplarily, such selected from the group consisting of a halogen (in particular Cl⁻, Br⁻, I⁻ and/or F⁻), OH⁻, H₂PO₄⁻, HPO₄²⁻, PO₄³⁻, SO₄²⁻, an anion of an organic acid (e.g., acetate, methanoate, propionate, a salt of a fatty acid, gluconate, lactate, citrate, etc.), an organic sulfonate, an organic sulfate, and organic phosphate.

An undesired local subcutaneous fat deposit in a mammal may be understood in the broadest sense as any unwanted accumulation in the mammal's body that bears a considerable content of adipose tissue (fatty tissue), i.e., a tissue comprising adipocytes (fat cells). In this context, the terms "fat deposit" and "fat pad" may be understood interchangeably. Preferably, in such tissue, adipocytes constitute for at least 25%, more preferably at least 50%, of the tissue volume. It will however be understood by the person skilled in the art that also the fat deposit will typically comprise other cell types such as cell types forming blood vessels, fibroblasts, blood cells and immune cells. The fat deposit to be treated is located subcutaneously, i.e., beneath the outer skin (deep dermis). The fat deposit of interest is typically located in the subcutis (hypodermis, hypoderm, subdermis).

The subcutaneous fat deposits are normally local fat deposits. Therefore, the present invention focuses on treating or preventing one or more subcutaneous fat deposit(s) in a particular part of the mammal's body rather than on a systemic treatment of disseminated or general obesity. These specific local subcutaneous fat deposit are often undesired and recalcitrant to diet and exercise. Therefore, it is intended to remove said fat deposit or at least reduce said fat deposit is size.

According to the present invention, the compound comprised in the composition of the present invention is directly injected into the undesired local subcutaneous fat deposit *in situ.* Therefore, the composition of the present invention is directly brought in contact with the respective adipose tissue. A first pass effect obtained when administering the composition of the present invention orally is therefore circumvented. Further, systemic side effects observable when administering such composition systemically (e.g, orally, nasally, intravenously (i.v.), intraarterially (i.a.), intraperitoneally (i.p.), intramusculary (i.m.)) are also avoided. Therefore, comparably low systemic side effects, but rather site-specific (i.e., local) effects on the particular undesired subcutaneous fat deposit are observed.

Injection into the undesired local subcutaneous fat deposit may be injection once ((acute) single administration) or may be repeated injection. Repeated injection may exemplarily be injection two times, three times, four times, five times, six times, seven times, eight times, nine times, ten times, or more than ten times. Preferably, injection is made 1-6 times. Therefore, the injection may be exemplarily an injection only once, two injections, three injections, four injections, five injections, or six injections.

As used herein, each injection means application within one session, i.e., typically within less than one hour. The person skilled in the art will note that a single injection may optionally include several punctures of a single local fat deposit from different sides and/or angles and/or of more than one local fat deposits within a single session. Each puncture is a penetration of the mammal's skin with an injection tool, such as a needle (acus) accompanied by applying the composition of the present invention through said injection tool to the local fat deposit punctured. Exemplarily, each local fat deposit may be punctured once, may be punctured twice, may be punctured 3 times, may be punctured 4 times, may be punctured 5 times, may be punctured 6 times, or may be punctured more than 6 times at different sides and/or from different angles.

Fat deposits may be punctured more than once in a single session. Then, preferably, the distance between two punctures (spacing of the punctures) is preferably at least 0.25 cm, more preferably at least 0.5 cm, in particular at least 1 cm or even more than 2 cm such as at least 3 cm, 4 cm or at least 5 cm. In other words, there are preferably not more than 4 punctures per square centimeter (cm²), more preferably not more than 2 punctures per cm², even more preferably not more than 1 punctures per cm² or even less such as not more than 0.5 or not more than 0.25 or not more than 0.1 punctures per cm². Exemplarily, for treating a double chin, up to 20 single punctures may be used in one session.

Between two injection sessions, there may be a time interval of less than 24 hours, between one and seven days, between one week and two weeks, between two weeks and a month, between a month and six months, between six months and a year. Injective administration may be daily, every second day, every three days, weekly, biweekly, monthly, twice a year, yearly, or less often. Preferably, between two injections, there may be a time interval of 1-4 weeks. Exemplarily, the time interval may be in the range of from 5-9 days, 9-16 days, 16-23 days, 23 to 30 days.

More preferably, an undesired fat deposit in a mammal is injected once (a single injection session) or 2 to 6 times (2-6 injection sessions) with a time interval between two injections of 1 to 4 weeks. Clinically viable administration schemes may be determined by the person skilled in the art based on balancing efficacy and toxicity. Injection may exemplarily be injection with a syringe.

The volume of the liquid or semi-solid (i.e., viscous) composition of the present invention injected into the mammal per injection may optionally be in the range of several microliters to several milliliters. Exemplarily, the amount injected at once in a single injection may be in the range of 50-150 µl, in the range of 150-300 µl, in the range of 300-500 µl, in the range of 400-600 µl, in the range of 500-1000 µl or in the range of 1-2 ml. The person skilled in the art will notice that the volume of the liquid or semi-liquid composition injected into the mammal per injection will be adjusted to the size of the fat deposit and the concentration of the active compound in the composition.

As used throughout the present invention, the term "mammal" may be understood in the broadest sense as any mammalian animal including humans, irrespective whether clinical symptoms occur or do not occur. Preferably, the mammal is a human or a domestic animal such as an animal selected from the group consisting of mouse, rat, cow, pig, dog, cat, horse. Most preferably, the mammal is a human (patient).

Particularly preferably, the composition of the present invention is a pharmaceutical composition.

The at least one further component C may be any component that is pharmaceutically acceptable, in particular pharmaceutically acceptable for being injected into a local fat deposit. Exemplarily, at least one further component C may be selected from one or more pharmaceutically acceptable carrier(s), one or more detergent(s) and one or more pharmaceutically acceptable additive(s). Such components are further exemplified below.

The person skilled in the art will adjust the dose injected in a single shot in view of the size of the undesired subcutaneous fat deposit to be treated or prevented, the body weight and health status of the mammal and the administration scheme chosen.

In a preferred embodiment, injecting said composition into the local subcutaneous fat deposit is for reducing said local subcutaneous fat deposit in size for body contouring, in particular via adipocytolysis.

Reducing is size may be understood in the broadest sense as any decrease of the volume of the undesired local subcutaneous fat deposit. Reducing in size may be a decrease of the volume of the undesired local subcutaneous fat deposit in which the compound comprised in the composition of the present invention is injected by at least 5 %, at least 10 %, at least 25 %, at least 50 %, at least 75 %, at least 90 %, or at least 95 % in volume.

A large variety of conditions associated with an undesired local subcutaneous fat deposit are known in the art.

In a preferred embodiment, the condition associated with an undesired local subcutaneous fat deposit is selected from the group consisting of lipomas, fat redistribution syndrome, lipodystrophy, lower eyelid fat herniation, fat deposits associated with cellulite, Dercum's disease, dorsocervical fat, double chin, submental fat, bra rolls, visceral adiposity, local hyperadiposity, diffused body fat around trunk and arms, and a condition associated with breast reduction.

Such condition may be associated with a rather pathological condition (e.g., lipomas (International Statistical Classification of Diseases and Related Health Problems 10th Revision (ICD-10) of 2015, class D17), Dercum's disease (Lipomatosis dolorosa, ICD-10 class E88.2), lipodystrophy (ICD-10 class E88.1), fat redistribution syndrome, etc.) or be a rather aesthetic concern (e.g., lower eyelid fat herniation, fat deposits associated with cellulite, dorsocervical fat, visceral adiposity, local hyperadiposity, diffused body fat around trunk and arms, a condition associated with breast reduction, etc.). It will however be understood that, in some cases, pathological conditions and aesthetic concerns are hardly distinguishable.

The composition may be any pharmaceutically acceptable composition suitable for being injected into a fat deposit comprising the compound of formula (I). Preferably, the composition comprises the compound of formula (I) or pharmaceutically acceptable salt thereof in an amount of 0.01 to 10 % by weight, more preferably in an amount of 0.05 to 5 % by weight, even more preferably in an amount of 0.1 to 1.5 % by weight, in particular in an amount of 0.1 to 1 % by weight.

In a more preferred embodiment, in the composition, the composition is a buffered or unbuffered (in particular buffered), isotonic or hypertonic (in particular isotonic) aqueous or non-aqueous composition comprising the compound of formula (I) or pharmaceutically acceptable salt thereof in an amount of 0.1 to 1.5 % by weight. More preferably, the composition is a buffered, isotonic aqueous or non-aqueous composition comprising the compound of formula (I) or pharmaceutically acceptable salt thereof in an amount of 0.1 to 1.0 % by weight.

Preferably, the compound is amphiphilic. Therefore, the compound may comprise a rather hydrophilic (hydrophobic) and a rather lipophilic (hydrophobic) part. Therefore, then, the compound may bear detergent-like properties and may mediate and support the formation and/or stabilization of emulsions between lipophilic and hydrophilic components (e.g., an oil-in-water emulsion). The compound may be amphiphilic due to one or more partial charges or charges. Preferably, the amphiphilic compound may bear one (i.e. a single) or more charged group(s).

More preferably, the compound bears one charged group. This charged group may be charged one-fold (i.e., + or -), two-fold (i.e., 2+ or 2-), three-fold (i.e., 3+ or 3-) or more often. Preferably, the charged group is charged one-fold or two-fold, in particular one-fold. The charge may be positively or negatively. In particular, the charge is a one-fold negative charge. Preferably, the charge is located at residue R4.

Preferably, R1 is an unsubstituted C₁₋₃ alkyl residue, in particular an ethyl residue (-CH₂CH₃).

Preferably, R2 is hydrogen or -OH.

Preferably, R3 is hydrogen or -CH₃.

Preferably, R4 is a negatively charged group comprising not more than two carbon atoms. More preferably, R4 is -COO⁻X⁺ or -OSO₃⁻X⁺, wherein X⁺ is a proton or a pharmaceutically acceptable cation. R4 may also be an ester or an amide formed by reacting a carboxyl moiety bound to the compound of the present invention or an activated form thereof with a compound bearing a hydroxyl or amine group.

Herein, X⁺ may preferably but not necessarily be a one-fold positively charged ion. Exemplarily but not limiting, the cation may be selected from the group consisting of an alkali metal (in particular, Na⁺ and/or K⁺), an alkaline earth metal (in particular, Mg²⁺ and/or Ca²⁺), ammonium (NH₄⁺), Fe²⁺, Fe³⁺, Zn²⁺, and an organic amine. Preferably, the cation is a proton (H⁺) or an alkali metal (in particular, Na⁺ and/or K⁺).

Preferably, R5 is -OH.

In a more preferred embodiment, the residues in the compound of the present invention are defined as:
R1 is a C₁₋₃ alkyl residue;
R2 is hydrogen or -OH;
R3 is hydrogen or -CH₃;
R4 is a negatively charged organic group; and
R5 is -OH.

In a highly preferred embodiment, in the compound R1 is -CH₂CH₃ (ethyl residue) and/or R5 is -OH, in particular R1 is -CH₂CH₃ and R5 is -OH.

In a preferred embodiment, in the composition, the compound or pharmaceutically acceptable salt thereof has a structure according to formula (II) 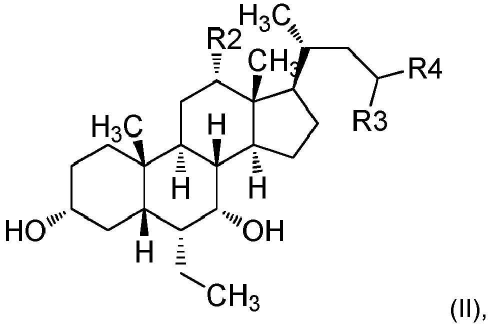 wherein:
R2 is hydrogen or -OH;
R3 is hydrogen or -CH₃; and
R4 is defined as above.

In the view of above, in a highly preferred embodiment, the residues in a compound according to the present invention are defined as follows:
R1 is -CH₂CH₃;
R2 is hydrogen or -OH;
R3 is hydrogen or -CH₃;
R4 is -COO-X⁺ or -OSO₃⁻X⁺, wherein X⁺ is a proton or a pharmaceutically acceptable cation; and
R5 is -OH.

In a particularly preferred embodiment, in the composition, the compound is selected from the group consisting of any of the structures according to formulae (III)-(V): 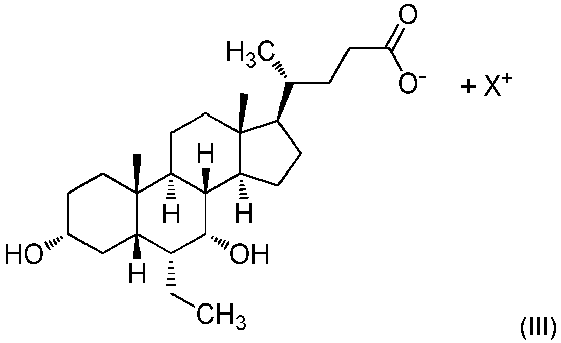 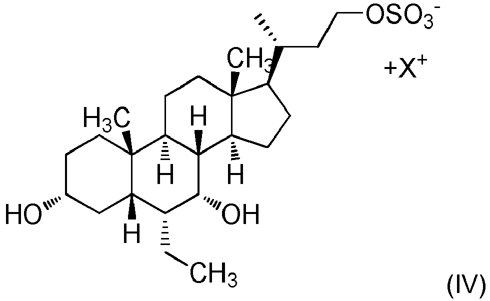 and 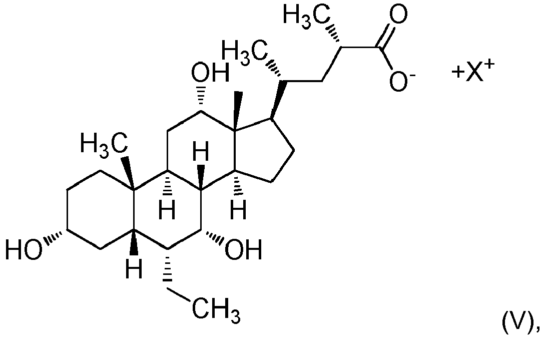 wherein X⁺ is a proton or a pharmaceutically acceptable cation.

In the composition, the compound may be a compound selected from:
(a) obeticholic acid ((3α,5β,6α,7α)-6-ethyl-3,7-dihydroxycholan-24-oic acid (6-ECDCA), (4R)-4-[(3R,5S,6R,7R,8S,9S,10S,13R,14S,17R)-6-ethyl-3,7-dihydroxy-10,13-dimethyl-2,34,5,6,7,8,9,11,12,14,15,16,17-tetradecahydro-1H-cyclopenta[a]phenanthren-17-yl]pbtanoic acid, 6α-ethyl-chenodeoxycholic acid, compound INT-747) (cf., compound of formula (III);
(b) compound INT-767 (cf., compound of formula (IV));
(c) compound INT-777 (cf., compound of formula (V)); and
(d) a mixture of two or al of (a), (b) and (c), thus a mixture of (a) and (b), of (b) and (c), of (a) and (c) or of (a), (b) and (c).

Such compounds may be obtained from a commercial supplier or may be synthesized. Exemplarily, obeticholic acid is as such well-known to a person skilled in the art. Semisynthetic bile acid analogs like the compounds laid out above are exemplarily known from WO 2005/082925, WO 2008/091540, WO 2008/002573 and Roda, 2014, J Pharmacol Exp Ther 350:56-68.

The compounds used in the context of the present invention bear a comparably high agonistic activity on farnesoid X receptor (FXR) and/or Takeda G protein-coupled receptor 5 (TGR5). In fact, the agonistic activity of a variety of such compounds is far higher than that of deoxycholate. Deoxycholate bears comparably high half-maximal effective concentrations (EC50 values) for FXR (50 µM) and for TGR5 (1.3 µM). In contrast, the compound of a structure of formula (III) (obeticholic acid) bears significantly lower EC50 values of for FXR (0.1 µM) and for TGR5 (0.1 µM), respectively. Also, the compound of a structure of formula (IV) bears significantly lower EC50 values for FXR (0.03 µM) and for TGR5 (0.1 µM). Further, the compound of a structure of formula (V) bears a significantly lower EC50 value for TGR5 (0.1 µM) while being not being particular effective against FXR. This evidences that the compounds particularly preferably used in the context of the present invention are particularly effective agonists of FXR and/or TGR5 and are therefore particularly beneficial when injected because their mode of activity is not restricted to their activity as detergent.

The composition of the present invention may in principle comprise the components (I) and C in any ratio.

Preferably, the composition consists of:
(A) 0.1 to 5 % (w/w) of one or more pentacyclic triterpenoid compounds or pharmaceutically acceptable salts thereof for use as laid out above or one or more pharmaceutically acceptable amino acid and/or acyl conjugates thereof;
(B) 45 to 99.9 % (w/w) of a pharmaceutically acceptable carrier, in particular a liquid pharmaceutically acceptable carrier comprising or consisting of one or more pharmaceutically acceptable buffers or solvents;
(C) 0 to 10 % (w/w) of one or more pharmaceutically acceptable detergents; and
(D) 0 to 40 % (w/w) of one or more pharmaceutically acceptable additives.

As used herein in the context of the contents of the components (A), (B), (C) and/or (D) in the composition, the percentage ranges refer to the contents of the respective component(s) in the composition as a whole (comprising or consisting of the components (A)-(D)).

More preferably, the composition consists of:
(A) 0.1 to 3 % (w/w) of one or more pentacyclic triterpenoid compounds or pharmaceutically acceptable salts thereof for use as laid out above or one or more pharmaceutically acceptable amino acid and/or acyl conjugates thereof;
(B) 67 to 99.9 % (w/w) of a pharmaceutically acceptable carrier, in particular a liquid pharmaceutically acceptable carrier comprising or consisting of one or more pharmaceutically acceptable buffers or solvents;
(C) 0 to 10% (w/w) of one or more pharmaceutically acceptable detergents; and
(D) 0 to 20 % (w/w) of one or more pharmaceutically acceptable additives.

Even more preferably, the composition consists of:
(A) 0.1 to 2 % (w/w) of one or more pentacyclic triterpenoid compounds or pharmaceutically acceptable salts thereof for use as laid out above or one or more pharmaceutically acceptable amino acid and/or acyl conjugates thereof;
(B) 73 to 99.9 % (w/w) of a pharmaceutically acceptable carrier, in particular a liquid pharmaceutically acceptable carrier comprising or consisting of one or more pharmaceutically acceptable buffers or solvents;
(C) 0 to 5% (w/w) of one or more pharmaceutically acceptable detergents; and
(D) 0 to 20 % (w/w) of one or more pharmaceutically acceptable additives.

In a preferred embodiment, the composition consists of:
(A) 0.1 to 2 % (w/w) of one or more compounds or pharmaceutically acceptable salts thereof for use according to any of claims 1 to 9, one or more pharmaceutically acceptable prodrugs thereof such as amino acid and/or acyl conjugates thereof, solvates thereof, and/or complexes thereof;
(B) 81 to 99.9 % (w/w) of a pharmaceutically acceptable carrier, in particular a liquid pharmaceutically acceptable carrier comprising or consisting of one or more pharmaceutically acceptable buffers or solvents;
(C) 0 to 2 % (w/w) of one or more pharmaceutically acceptable detergents; and
(D) 0 to 5 % (w/w) of one or more pharmaceutically acceptable additives.

In a preferred embodiment, the composition consists of:
(A) 0.1 to 2 % (w/w) of one or more compounds or pharmaceutically acceptable salts thereof for use according to any of claims 1 to 9, one or more pharmaceutically acceptable prodrugs thereof such as amino acid and/or acyl conjugates thereof, solvates thereof, and/or complexes thereof;
(B) 91 to 99.9 % (w/w) of a pharmaceutically acceptable carrier, in particular a liquid pharmaceutically acceptable carrier comprising or consisting of one or more pharmaceutically acceptable buffers or solvents;
(C) 0 to 2 % (w/w) of one or more pharmaceutically acceptable detergents; and
(D) 0 to 5 % (w/w) of one or more pharmaceutically acceptable additives.

In a more preferred embodiment, the composition consists of:
(A) 0.1 to 1.5 % (w/w) of one or more compounds or pharmaceutically acceptable salts thereof for use according to any of claims 1 to 9, one or more pharmaceutically acceptable prodrugs thereof such as amino acid and/or acyl conjugates thereof, solvates thereof, and/or complexes thereof;
(B) 91.5 to 99.9 % (w/w) of a pharmaceutically acceptable carrier, in particular a liquid pharmaceutically acceptable carrier comprising or consisting of one or more pharmaceutically acceptable buffers or solvents;
(C) 0 to 2 % (w/w) of one or more pharmaceutically acceptable detergents; and
(D) 0 to 5 % (w/w) of one or more pharmaceutically acceptable additives.

Due to the fact that the compound comprised in the composition of the present invention is intended to be injected into the undesired local subcutaneous fat deposit in a mammal, the composition is preferably administered to the mammal in liquid or pasty form. Therefore, the composition is preferably liquid or pasty. Particularly preferably, the composition is administered to the mammal in liquid form. Therefore, the composition is particularly preferably liquid. It will however be understood that the composition may optionally also be stored and/or delivered in solid state and then be dissolved, suspended or emulsified just prior to use. This may increase shelf life thereof.

A pharmaceutically acceptable carrier according the present invention may be any additive that is pharmaceutically acceptable, therefore, any additive that is nontoxic to the mammal. As the composition is preferably liquid or pasty, in particular liquid, the pharmaceutically acceptable carrier preferably comprises or consists of one or more solvents such as, e.g., water, an aqueous buffer (e.g., a saline or phosphate buffered saline), dimethyl sulfoxide (DMSO), ethanol, vegetable oil, paraffin oil or combinations thereof.

The composition of the present invention comprises at least one compound (I) of the present invention. Optionally, the composition may also comprise more that one compound of the present invention such as the combination of two, three, four, five or even more compounds of the present invention.

Furthermore, a pharmaceutically acceptable carrier may optionally further contain one or more detergent(s) such as, e.g., sodium lauryl sulfate (SLS)/ sodium doceyl sulfate (SDS).

Furthermore, a pharmaceutically acceptable carrier may optionally further contain one or more pharmaceutically acceptable additives. Such pharmaceutically acceptable additives may exemplarily be selected from the group consisting of one or more coloring agent(s) (e.g., TiO₂, food coloring), one or more vitamin(s), one or more salt(s) (e.g., sodium, potassium, magnesium, calcium, and/or zinc salts), one or more humectant(s) (e.g., sorbitol, glycerol, mannitol, propylene glycol, polydextrose), one or more enzyme(s), one or more preserving agent(s) (e.g., benzoic acid, methylparabene), one or more texturing agent(s) (e.g., carboxymethyl cellulose (CMC), polyethylene glycol (PEG), sorbitol), one or more emulsifier(s), one or more separating agent(s), one or more antioxidant(s), one or more herbal and plant extract(s), one or more stabilizing agent(s), one or more polymer(s) (e.g., hydroxypropyl methacrylamide (HPMA), polyethylene imine (PEI), carboxymethyl cellulose (CMC), polyethylene glycol (PEG)), one or more uptake mediator(s) (e.g., polyethylene imine (PEI), dimethyl sulfoxide (DMSO), a cell-penetrating peptide (CPP), a protein transduction domain (PTD), an antimicrobial peptide, etc.) one or more antibody/antibodies, one or more counterstain dye(s) (e.g., fluorescein, fluorescein derivatives, Cy dyes, an Alexa Fluor dye(s), S dye(s), rhodamine, quantum dot(s), etc.), and one or more homeopathic ingredient(s).

The composition may or may not comprise one or more natural bile acids (e.g., deoxycholate (DC), chenodeoxycholis acid (CDCA), ursodeoxycholate (UDCA) and/or lithocholic acid (LCA)) and/or phosphatidylcholine (PC). Preferably, the composition of the present invention is free or at least essentially free of deoxycholate (DC), chenodeoxycholis acid (CDCA), ursodeoxycholate (UDCA), lithocholic acid (LCA) and phosphatidylcholine (PC). Should one or more of the aforementioned agent be present in the composition, this agent can be used in lower amounts due to the presence of the compound comprised in the composition of the present invention.

The present invention also relates to a method of treating or preventing a condition associated with an undesired local subcutaneous fat deposit in a mammal, wherein a sufficient amount of a composition according to the present invention comprising or consisting of:
(A) 0.1 to 5 % (w/w) of one or more pentacyclic triterpenoid compounds or pharmaceutically acceptable salts thereof for use according to the present invention, one or more pharmaceutically acceptable prodrugs thereof such as amino acid and/or acyl conjugates thereof, solvates thereof, and/or complexes thereof;
(B) 85 to 99.9 % (w/w) of a pharmaceutically acceptable carrier, in particular a liquid pharmaceutically acceptable carrier comprising or consisting of one or more pharmaceutically acceptable buffers or solvents;
(C) 0 to 5 % (w/w) of one or more detergents; and
(D) 0 to 5 % (w/w) of one or more pharmaceutically acceptable additives,
   is administered to said mammal by injecting said compound into said undesired local subcutaneous fat deposit *in situ.*

In this context, it will be understood that all definitions provided above apply *mutatis mutandis* to this aspect of the present invention.

The compound comprised in the composition of the present invention may be embedded into a pharmaceutically acceptable structure that is suitable for injections such as, e.g., a dispersion, in particular an emulsion (e.g., an oil-in-water (O/W) emulsion, a water-in-oil (W/O) emulsion, a water-in-oil-in water (W/O/W) emulsion, or an oil-in-water-oil emulsion (O/W/O), in particular a micelle or a liposome structure), a suspension of beads (e.g., beads comprising said compound in the interior and/or in a shell structure and/or attached to the bead surface, wherein the beads may exemplarily be microbeads and/or nanobeads), a liquid or semi-solid (i.e., viscous or gel-like) solution or suspension wherein said compound is dissolved/suspended, a liquid or semi-solid composition comprising fibers (e.g., a hydrogel) in which said compound is incorporated and/or attached to, soluble, partly soluble or insoluble biocompatible polymer strands to which the compound is attached to (e.g., hydroxypropyl methacrylamide (HPMA), polyethylene imine (PEI), carboxymethyl cellulose (CMC), polyethylene glycol (PEG), polylactic acid (PLA), polyethylene imine (PEI), polypeptides, polysaccharides (e., dextran), or a combination of two or more thereof), or a combination of two or more thereof.

In a preferred embodiment, the compound or pharmaceutically acceptable salt thereof for use is embedded into a liposome structure.

As indicated above, the present invention also refers to cosmetic and medicinal aesthetic uses of the compound comprised in the composition of the present invention.

Therefore, in a further aspect, the present invention refers to a method for cosmetic reduction of an undesired local subcutaneous fat deposit, comprising the step of locally injecting a sufficient amount of a composition according to the present invention into said local subcutaneous fat deposit.

In this context, it will be understood that all definitions provided above apply *mutatis mutandis* to this aspect of the present invention. The cosmetic and medicinal aesthetic use can be any use associated with an undesired local subcutaneous fat deposit known in the art. In a preferred embodiment, the cosmetic method is used for body contouring.

Therefore, the outer shape and appearance of the mammal's body may be changed and potentially improved in that the one or more undesired local subcutaneous fat deposit(s) are (optically) removed or at least reduced in size.

The medicinal aesthetic/cosmetic method may be used for all the purposes as indicated above when there is a medicinal aesthetic/cosmetic need thereof.

Therefore, in a preferred embodiment, the undesired local subcutaneous fat deposit is selected from the group consisting of lower eyelid fat herniation, double chin, submental fat, bra rolls, fat deposits associated with cellulite, diffused body fat around trunk and arms, and a condition associated with breast reduction, lipomas, fat redistribution syndrome, lipodystrophy, Dercum's disease, dorsocervical fat, and local hyperadiposity.

A still further aspect of the present invention relates to the Use of a compound or a pharmaceutically acceptable salt thereof according to the present invention for body contouring by means of adipocytolysis.

In this context, it will be understood that all definitions provided above apply *mutatis mutandis* to this aspect of the present invention.

The invention is further explained by the following examples and claims.

### Brief Description of the Figures

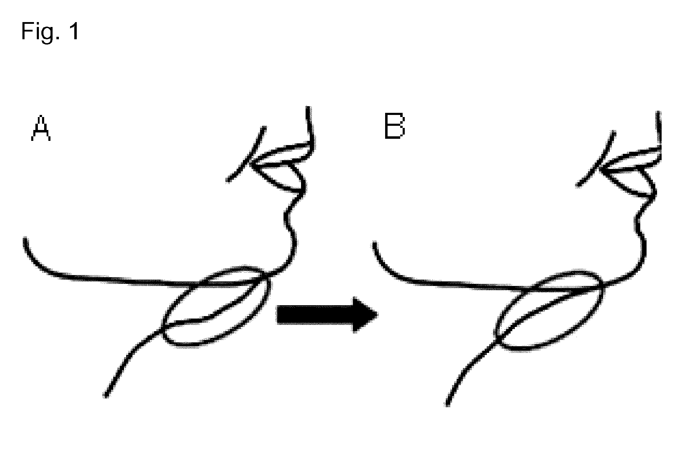

Figure 1 schematically shows an example of the technical effect of the composition of the present invention, i.e., the treatment of a double chin. A: before the treatment, B: after the treatment.

### Examples

### In vitro use of the compounds of the present invention

The following experimental setup was used for determination of adipocytolytic potential of test compounds with preadipocytes and mature adipocytes:
Preadipocytes (PromoCell) were seeded in Preadipocyte Growth Medium at 45000 cells/ cm² to allow attachment overnight. Cells were cultured at 37°C and 5% CO₂ untill postconfluence. Differentiation was started with incubation of cells in Differentiation Medium for 7 days followed by incubation in Nutrition medium for 14 further days. After differentiation process, mature adipocytes were incubated with indicated concentration of indicated triterpenes.

6 replicates of each test compound concentration were tested. Each plate included a negative control and a compound interference control (for all concentrations, duplicates).

Cell viability as surrogate for adipocytolytic potential of compounds were analysed by alamar blue assay. In brief, 20µl of a 0,1mg/ml Resazurin sodium salt (Sigma; R7017-1G) solution was added to each well 48 hrs after compound incubation. The cells were incubated for 90 min at 37°C and 5% CO₂ and the resulting conversion of resazurin to resorufin was measured by a fluorescence plate reader at 590 nm. Vehicle controls were set as 100%. IC50-values were analysed using SigmaBlotV13.

For analysing the potential of compounds to induce apoptosis the Caspase-Glo® 3/7 Assay was used (Promega) as recommended by the supplier. In brief, 100µl of Caspase Reagent was added to 100µl cell culture medium and cells were incubated at 37°C and 5% CO₂ for 1 hr. Luminescence signal was measured with a SynergyH4 plate reader. Vehicle controls were used to define basal apoptosis as 100%.

**Table 1 Impact on viability of in vitro matured adipocytes after 48h incubation with indicated compound in increasing concentration**

| | Viability Adipocytes (%) | |
|---|---|---|
| conc (µM) | Deoxycholic acid | Obeticholic acid |
| 10 | 106.98 | 101.15 |
| 50 | 95.61 | 89.63 |
| 100 | 94.30 | 49.17 |
| 300 | 22.06 | 0.29 |
| 600 | 0.11 | - |

**Table 2 Impact on viability of pre-adipocytes after 48h incubation with indicated compound in increasing concentration**

| | Viability Preadipocytes (%) | |
|---|---|---|
| conc (µM) | Deoxycholic acid | Obeticholic acid |
| 10 | 96.22 | 92.95 |
| 50 | 95.61 | 83.06 |
| 100 | 98.03 | 21.37 |
| 300 | 26.08 | 0.41 |
| 600 | 0.00 | |

**Table 3 IC50-values of obeticholic acid and deoxycholic acid**

| Compound | IC50 [µM] | IC50 [µM] |
|---|---|---|
| | Adipocytes | Pre-ad ipocytes |
| Obetichol ic acid | 93.9 | 87.25 |
| Deoxycholic acid | 243.60 | 232.40 |

Obeticholic acid shows a dose dependent decrease of viability on preadipocytes and adipocytes. The IC50-value of Obeticholic acid (93,9 µM) is about 2.5fold lower than the one of Deoxycholic acid (243,6 µM). This demonstrates a 2.5 times higher adipocytolytic activity of Obeticholic acid compared to Deoxycholic acid.

**Table 4 Impact on apoptosis induction of adipocytes after 6hrs and 48hrs with indicated compound concentrations of obeticholic acid**

| | Apoptosis Adipocytes (%) | |
|---|---|---|
| conc (µM) | Obeticholic acid 6 hrs | Obeticholic acid 24 hrs |
| 50 | 132.28 | 146.44 |
| 100 | 503.28 | 180.88 |
| 300 | 1164.30 | 779.64 |

Obeticholic acid shows a concentration dependent increase of apoptosis induction with max. (11.6 fold over basal level) at 300µM after 6hrs. Extended incubation time leads to concentration dependent lower measurable apoptotic induction values.

In summary, data show a clear apoptosis induction capacity of Obeticholic acid.

Obeticholic acid serving as an example for semisynthetic bile acids analogs provides surprisingly good results in acting against adipocytes. In comparison to deoxycholate, obeticholic acid bears a superior effect on adipocytes. At a concentration of 100 µM of obeticholic acid, the vitality of adipocytes decreases to approximately 50% whereas a concentration as high as 200 µM (i.e., the double concentration) of deoxycholate is required to achieve such 50%-decrease in vitality. Obeticholic acid further reveals as being pro-apoptotic. Therefore, reducing the vitality of adipocytes by means of obeticholic acid notably and unexpectedly follows a milder mechanism of action (induction of apoptosis) compared to the employment of deoxycholate (leading to necrosis). In brief, obeticholic acid is more effective and provokes lower side effects compared to deoxycholate.

### In vivo use of the compounds of the present invention

### Chemicals

### Obeticholic acid, Na₃PO₄, glycerol.

### Formulation preparation

All work was done under sterile extractor hood/luminar airflow working environment. Final solution were filtered through sterile 0.22um Millipore PES membrane syringer filter unit. Preparation of the solution:
(1) Obeticholic acid injection solution.

To 0.4099g Na₃PO₄ water for injection was added to a final weight of 50 g.

For the highest (2%) concentration, 100 mg of obeticholic acid were added with 2.75 ml of above solution, 112,5 mg of glycerol and finally 2.038 ml of sterile water to make 5 ml solution. Lower concentrations were adjusted accordingly.

### Animals

Male Sprague-Dawley rats (200-300g of body weight) were used for this study (Vital River Laboratories Animal Ltd., China) and were housed in a temperature (22 ± 3 °C) and humidity (55 ± 15 %) controlled AAALAC accredited facility. Animals were provided with food (Keaoxieli certified Rodent Diet) and sterile water *ad libitum.* The animals were on a 12-hour light/dark (6:00 am /6:00 pm) cycle and acclimated for 1 week prior to the study. Rats were housed in a plexiglas cage (3 rats per cage).

### Administration

After shaving the skin the inguinal area, rats, were injected into the inguinal fat pads (3 injection sites per fat pad for every rat, 100ul/per site). For all groups, 6 rats were injected with sterile vehicle, or obeticholic acid on day 1, day 7 and day 14 in the right pad and left fat pad was intact. For the other 6 rats were injected with sterile vehicle obeticholic acid on day 1, day 7 and day 14 in the left pad and right pad was intact.

### Dose selection

Obeticholic acid was injected at the concentration of 0.5, 1 and 2 %.

### Inguinal fat pad volume measurement

Isolated inguinal fat pad volume was measured using YLS - 7 b digit volume measuring instrument (Jinan YiYan Technology Development co., LTD, Shandong, P.R. China) according to manufacturer instructions.

### Fat content analysis (8 rats per group)

Rats were euthanized and the abdomen was cut and opened. Inguinal fat pads were exposed and a photo was taken. Then inguinal fat pads were isolated, and a gross photo was taken again followed by weighting of the pad. Total fat lipids were extracted from the inguinal fat using a protocol adapted from Folch et al. Fresh fat tissue (1g) was homogenized in 20ml of a 2:1 chloroform-methanol solution. The homogenate was filtered using a fat-free filter paper and funneled into a pre-weighted 50-ml glass vial. Then the previous vial and funnel were washed with an additional 5ml of a 2:1 chloroform-methanol solution. Afterwards 2.5 ml of 0.9% NaCl was added. The lipids were separated by centrifugation at 1,800 g, 10°C for 5 min; the aqueous layer was discarded and the tube was flushed with nitrogen until the lipid pellet was dry. The tube containing the lipid pellet was reweighed, and total lipid extracted per gram of total tissue was calculated.

Reference: Folch J., A simple method for the isolation and purification of total lipides from animal tissues. J Biol Chem 226:497-509, 1957.

### Fat histology analysis (4 rats per group)

Inguinal fat embedded in paraffin were cross-sectioned (5 µm each). For each mouse, a serial section at intervals of 40 to 50 µm was made and one section was collected for every 10 sections. In total, five sections per animal were used for Hematoxylin and eosin (H&E) stain and adipocyte number and size analysis. Histologic examination was performed by well-trained pathologists in a double-blind fashion. Three factors were examined in each sample: the quantity of normal fat, fat necrosis and inflammatory activity. Each category was scored in semiquantitative fashion into one of five grades according to the percentage in one microscopic field (×100): grade 0 (0%), grade 1 (<25%), grade 2 (26% to 50%), grade 3 (51% to 75%), and grade 4 (76% to 100%). H&E staining was used in evaluation of the inflammatory status.

### Statistical Analysis

Results were expressed as mean ± SEM. Statistical analysis was performed using ANOVA on ranks, followed if significant by Mann-Whitney test. The difference was considered significant when p< 0.05.

The effect of obeticholic acid on inguinal fat pad in rats was investigated by injecting at 3 sites 100 µl each on days 1, 7, 14 and analysis on day 35. The results are depicted in Table 1. Herein, the values representmean ±SEM. *-P<0.05 vs control.

**Table 5 Effect of obeticholic acid on inguinal fat pad in rats.**

| Obeticholic acid concentration (%) | Inguinal fat pad weight difference to contralateral (sham) pad (g) N=12 | Inguinal fat pad volume difference to contralateral (sham) pad (g) N=12 | Inguinal fat pad fat content difference to contralateral (sham) pad (g) N=8 |
|---|---|---|---|
| 0 (control) | 0.004±0.03 | 0.017±0.02 | -0.023±0.03 |
| 0.5 | -0.192±0.04* | -0.186±0.04* | -0.169±0.02* |
| 1 | -0.352±0.03* | -0.355±0.03* | -0.387±0.04* |
| 2 | -0.526±0.07* | -0.541±0.08* | -0.523±0.05* |

The results demonstrate that obeticholic acid is a highly effective compound for injection-based body contouring and is active in a concentration-dependent manner.

Histology analysis revealed that treatment with obeticholic acid produced an increase in inflammation at 2 % but not at 1 or 0.5%. Other parameters like fat necrosis and normal fat content as measured by histology were not affected.

## Claims

1. A composition comprising a compound or a pharmaceutically acceptable salt thereof having a structure according to formula (I) 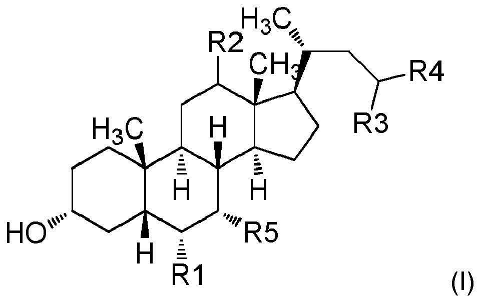 wherein:
R1 is a C₁₋₄ alkyl residue;
R2 is hydrogen or -OH;
R3 is hydrogen or a C₁₋₄ alkyl residue;
R4 is selected from the group consisting of a charged group, preferably a negatively charged group, in particular -COO⁻X⁺ or -OSO₃⁻X⁺, wherein X⁺ is a proton or a pharmaceutically acceptable cation, a conjugate of a carboxylic group, in particular a glycin or taurine conjugate, and an isostere of -COO⁻X⁺; and
R5 is hydrogen or -OH;
and at least one further component C
for use in a method for local treating or preventing a condition associated with an undesired local subcutaneous fat deposit in a mammal, wherein said compound is injected into said local subcutaneous fat deposit.

2. The composition for use according to claim 1, wherein injecting said composition into the local subcutaneous fat deposit is for reducing said local subcutaneous fat deposit in size for body contouring, in particular via adipocytolysis.

3. The composition for use according to claim 1 or 2, wherein the condition associated with an undesired local subcutaneous fat deposit is selected from the group consisting of lipomas, fat redistribution syndrome, lipodystrophy, lower eyelid fat herniation, fat deposits associated with cellulite, Dercum's disease, dorsocervical fat, double chin, submental fat, bra rolls, local hyperadiposity, diffused body fat around trunk and arms, and a condition associated with breast reduction.

4. The composition for use according to any of claims 1 to 3, wherein the composition is a buffered, isotonic aqueous composition comprising the compound of formula (I) or pharmaceutically acceptable salt thereof in an amount of 0.1 to 1.5 % by weight.

5. The composition for use according to any of claims 1 to 4, wherein in the compound:
R1 is a C₁₋₃ alkyl residue;
R2 is hydrogen or -OH;
R3 is hydrogen or -CH₃;
R4 is a negatively charged organic group; and
R5 is -OH.

6. The composition for use according to any of claims 1 to 5, wherein in the compound R1 is -CH₂CH₃ and/or R5 is -OH.

7. The composition for use according to any of claims 1 to 6, wherein the compound or pharmaceutically acceptable salt thereof has a structure according to formula (II) 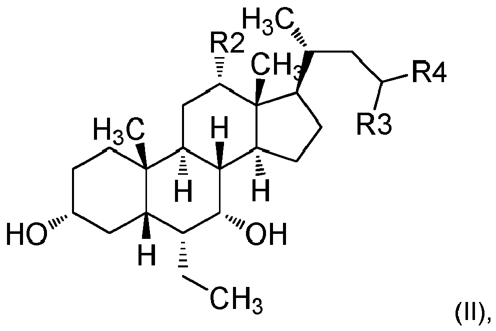 wherein:
R2 is hydrogen or -OH;
R3 is hydrogen or -CH₃; and
R4 is defined as in claim 1, in particular -OH.

8. The composition for use according to any of claims 1 to 7, wherein in the compound:
R1 is -CH₂CH₃;
R2 is hydrogen or -OH;
R3 is hydrogen or -CH₃;
R4 is -COO⁻X⁺ or -OSO₃⁻X⁺, wherein X⁺ is a proton or a pharmaceutically acceptable cation; and
R5 is -OH.

9. The composition for use according to any of claims 1 to 8, wherein the compound is selected from the group consisting of any of the structures according to formulae (III)-(V): 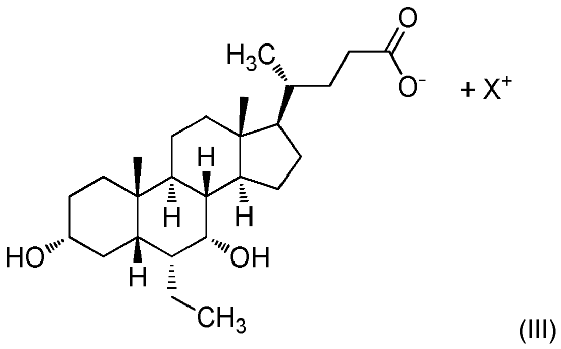 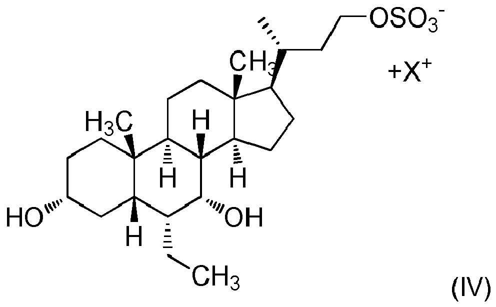 and 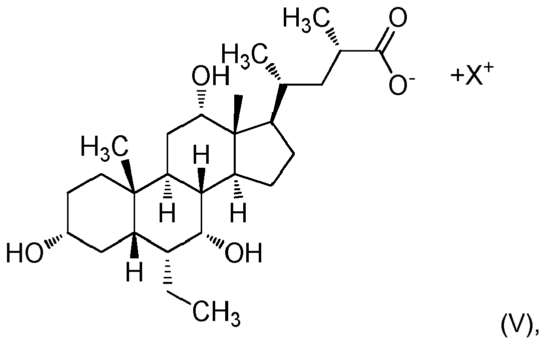 wherein X⁺ is a proton or a pharmaceutically acceptable cation.

10. The composition for use according to any of claims 1 to 9, wherein said composition consists of:
(A) 0.1 to 2 % (w/w) of one or more compounds or pharmaceutically acceptable salts thereof for use according to any of claims 1 to 9, one or more pharmaceutically acceptable prodrugs thereof, such as amino acid and/or acyl conjugates thereof, solvates thereof, and/or complexes thereof;
(B) 91 to 99.9 % (w/w) of a pharmaceutically acceptable carrier, in particular a liquid pharmaceutically acceptable carrier, comprising or consisting of one or more pharmaceutically acceptable buffers or solvents;
(C) 0 to 2 % (w/w) of one or more pharmaceutically acceptable detergents; and
(D) 0 to 5 % (w/w) of one or more pharmaceutically acceptable additives.

11. The composition for use according to any of claims 1 to 10, wherein the compound or pharmaceutically acceptable salt thereof for use is embedded into a liposome structure.

12. A method for cosmetic reduction of an undesired local subcutaneous fat deposit, comprising the step of locally injecting a sufficient amount of a composition according to any of claims 1 to 11 into said local subcutaneous fat deposit, in particular wherein said method is used for body contouring.

13. The method according to claim 12, wherein the undesired local subcutaneous fat deposit is selected from the group consisting of lower eyelid fat herniation, double chin, submental fat, bra rolls, fat deposits associated with cellulite, diffused body fat around trunk and arms, and a condition associated with breast reduction, lipomas, fat redistribution syndrome, lipodystrophy, Dercum's disease, dorsocervical fat, and local hyperadiposity.

14. Use of a compound or a pharmaceutically acceptable salt thereof according to any of claims 1 to 11 for body contouring by means of adipocytolysis.
